# EUROPÄISCHE PATENTSCHRIFT

(11) **EP 1 341 568 B1**
(45) Veröffentlichungstag und Bekanntmachung des Hinweises auf die Patenterteilung: **17.01.2007**
(21) Anmeldenummer: 01980544.9
(22) Anmeldetag: 06.11.2001
(51) Int. Cl.: A61M 1/10, H02K 1/12, H02K 7/14, H02K 3/47, H02K 5/04, H02K 15/14

(54) **MINIATURMOTOR**
MINIATURE MOTOR
MICROMOTEUR

(30) Priorität: 25.11.2000 DE 10058669
(43) Veröffentlichungstag der Anmeldung: 10.09.2003
(73) Patentinhaber: Abiomed Europe GmbH, 52074 Aachen (DE)
(72) Erfinder: Sieß, Thorsten, 52146 Würselen (DE); Döpper, Gebhard Dr., 74549 Wolpertshausen (DE)
(74) Vertreter: Selting, Günther
(86) Internationale Anmeldenummer: PCT/EP2001/012817
(87) Internationale Veröffentlichungsnummer: WO 2002/041935

(56) Entgegenhaltungen:
- EP-A- 0 764 448
- US-A- 4 482 829
- US-A- 4 679 313
- US-A- 5 911 685

## Beschreibung

Die Erfindung betrifft einen Mikromotor mit einem Stator, der eine Hülse mit einer Erregerwicklung und einen Rückschlussmantel aus beabstandeten ferromagnetischen Ringen aufweist, und insbesondere einen Mikromotor, der mit einem Pumpenteil kombiniert ist und eine Flügelradpumpe bildet.

Im Bereich der Medizintechnik werden Blutpumpen benötigt, die in den Körper eines Patienten eingeführt werden und in einer Arterie oder im Herzen platziert werden, um die Herzfunktion zu unterstützen. Eine für den Betrieb im Herzen vorgesehene intrakardiale Blutpumpe ist beschrieben in DE 198 21 307. Die Blutpumpe weist einen Antriebsteil mit einem elektrischen Mikromotor und einen Pumpenteil auf. Sie hat einen maximalen Außendurchmesser von etwa 8 mm und eine rigide Länge von weniger als 35 mm. Für die kleinformatige Blutpumpe, die die erforderliche Förderleistung erbringt, ist eine hohe Pumpendrehzahl von mindestens 30 000 Umdrehungen pro Minute erforderlich, typischerweise von 60 000 Umdrehungen pro Minute.

Ein Mikromotor, der für den Betrieb einer Blutpumpe geeignet ist, ist beschrieben in WO 98/44619. Dieser Mikromotor hat einen Stator und einen fest magnetisierten Rotor. Der Stator enthält eine Erregerwicklung, die von einem Rückschlussmantel umgeben ist. Der Rückschlussmantel bündelt die von der Erregerwicklung erzeugten Magnetfeldlinien und konzentriert sie, so dass nur wenig magnetischer Fluss durch Streufelder verloren geht. Der Rückschlussmantel besteht aus Blechen, die elektrisch gegeneinander isoliert und zu einem rohrförmigen Stapel zusammengesetzt sind.

Der Wunsch, Pumpen mit einem Katheter durch Punktion in das Gefäßsystem eines Patienten einzuführen, ohne den Patientenkörper operativ öffnen zu müssen, führt zu einem Bedarf an immer kleineren Pumpen und Motoren, deren Durchmesser im Bereich von 4 mm liegen. Bei einem elektrischen Mikromotor steht bei so kleinen Abmessungen für den Rückschlussmantel nur sehr wenig Platz zur Verfügung. Der Rückschlussmantel kann dabei nur eine Wandstärke von wenigen zehntel. Millimetern haben. Ein Rückschlussmantel von so geringer Wandstärke kann aus Blechen nicht zusammengefügt werden. Andererseits ist ein Rückschlussmantel zweckmäßig, um Energieverlust durch Magnetfeldstreuung zu vermeiden.

Der Erfindung liegt die Aufgabe zugrunde, einen Mikromotor mit hoher Leistungsfähigkeit und geringen radialen Abmessungen zu schaffen. Die Lösung dieser Aufgabe erfolgt erfindungsgemäß mit den im Patentanspruch 1 angegebenen Merkmalen. Hiernach besteht der Rückschlussmantel aus einem einstückigen Körper, bei dem benachbarte Ringe durch mindestens eine Brücke verbunden sind. Der Rückschlussmantel ist also aus einem einstückigen Rohr hergestellt und die Ringe sind nicht, wie dies bei Blechen der Fall ist, vollständig gegeneinander elektrisch isoliert. Vielmehr werden sie durch die Brücken einerseits in Verbindung und andererseits auf Abstand gehalten. Die Aufteilung des Rückschlussmantels in Ringe ist erforderlich, um die Ausbildung von Wirbelströmen zu begrenzen. Üblicherweise sind die Ringe separat voneinander angeordnet und durch Isolierschichten voneinander elektrisch völlig getrennt. Bei dem erfindungsgemäßen Mikromotor sind die Ringe nicht völlig voneinander getrennt, jedoch bewirken die Brücken, die nur an einzelnen Stellen des Umfangs vorhanden sind, nicht die Entstehung wesentlicher Wirbelströme. Folglich kann der gesamte Rückschlussmantel aus einem zusammenhängenden Körper hergestellt werden. Der Rückschlussmantel kann eine Wandstärke von 0,2 - 0,4 mm haben, also eine extrem geringe Wandstärke, wobei die die Ringe verbindenden Brücken für den erforderlichen Zusammenhalt und die notwendige Festigkeit sorgen. Durch die geringe Wandstärke des Rückschlussmantels kann es vorkommen, dass der Rückschlussmantel in der magnetischen Sättigung betrieben wird, ohne den gesamten Magnetfluss aufnehmen zu können. Der erfindungsgemäße Mikromotor hat eine Drehzahl von mindestens 30 000 Umdrehungen pro Minute und typischerweise von 60 000 Umdrehungen pro Minute. Der Rückschlussmantel hat die erforderliche strukturelle Festigkeit, die sowohl für die Montage als auch für den Betrieb des Mikromotors erforderlich ist. Bei Verklebung dünner Lamellen würde eine solche strukturelle Festigkeit nicht erreicht werden.

Gemäß einer bevorzugten Weiterbildung der Erfindung, bilden die Brücken des Rückschlussmantels einen längslaufenden durchgehenden Steg. Dies bedeutet, dass die Brücken relativ zueinander ausgerichtet sind. Diese Ausrichtung kann entlang einer zur Motorachse parallelen Linie erfolgen oder auch vorzugsweise entlang einer schraubenförmigen Linie. Vorzugsweise sind mindestens zwei Stege vorhanden, die umfangsmäßig verteilt angeordnet sind.

Gemäß einer bevorzugten Weiterbildung der Erfindung geht der Rückschlussmantel einstückig in ein Pumpengehäuse über. Der Rückschlussmantel, der im Bereich des Mikromotors in Ringe unterteilt ist, ist im Bereich des Pumpengehäuses durchgehend. Durch die Einstückigkeit von Pumpengehäuse und Rückschlussmantel wird eine besonders exakte Zentrierung des Pumpengehäuses relativ zum Mikromotor erreicht. Die Montage und die an sie zu stellenden Präzisionsanforderungen werden vereinfacht.

Die Erfindung betrifft ferner ein Verfahren zum Herstellen eines Mikromotors. Bei diesem Verfahren wird der Rückschlussmantel des Mikromotors aus einem ferromagnetischen Rohr durch Laserschneiden hergestellt, wobei umlaufende, durch Brücken unterbrochene Schlitze erzeugt werden.

Im Folgenden wird unter Bezugnahme auf die Zeichnungen ein Ausführungsbeispiel der Erfindung näher erläutert.

Es zeigen:
- Fig. 1: eine Seitenansicht einer Pumpe mit Mikromotor,
- Fig. 2: einen Längsschnitt durch Fig. 1, und
- Fig. 3: einen Querschnitt entlang der Linie III-III von Fig. 2.

Die in den Figuren dargestellte Pumpe weist einen Mikromotor 10 auf, an den sich ein Pumpenteil 11 axial anschließt. Der Pumpenteil 11 weist ein Flügelrad 12 auf, welches auf einer Welle 13 befestigt ist und in einem rohrförmigen Pumpengehäuse 14 rotiert.

Der Mikromotor 10 enthält einen Stator 15 und einen mit der Welle 13 verbundenen Rotor 16. Der Stator 15 besteht aus einer rohrförmigen Hülse 17 und einem die Hülse 17 eng umgebenden Rückschlussmantel 18. Der Rotor 16 enthält einen Magneten 19, dessen Nordpol N und Südpol S an diametral entgegengesetzten Stellen des Umfangs angeordnet sind. Der Magnet 19 ist auf der Welle 13 befestigt. Die Welle 13 ist am rückwärtigen Ende mit einem Kugellager 20 in der Hülse 17 gelagert und sie ist an dem dem Flügelrad 12 zugewandten vorderen Ende in einem Dichtlager 21 gelagert.

An das rückwärtige Ende des Stators 15 schließt sich ein Übergangsstück 22 an, das mit einem (nicht dargestellten) Katheter verbunden werden kann. Durch das Übergangsstück 22 verlaufen Drähte 23, die mit einer Erregerwicklung 24 im Innern der Hülse verbunden sind. Die Erregerwicklung 24 wird von einem extern gesteuerten Wechselstrom durchflossen, dessen Frequenz die Drehzahl des Motors bestimmt. Die Hülse 17, welche die Erregerwicklung 24 enthält, besteht aus einer etwa 0,2 mm starken Kunststoffschicht mit eingebetteten Drähten. Die Drähte sind zweilagig entsprechend einer vorgegebenen Konfiguration gewickelt. Zwischen dem Stator 15 und dem Rotor 16 besteht ein schmaler Spalt in der Größenordnung von einem zehntel Millimeter.

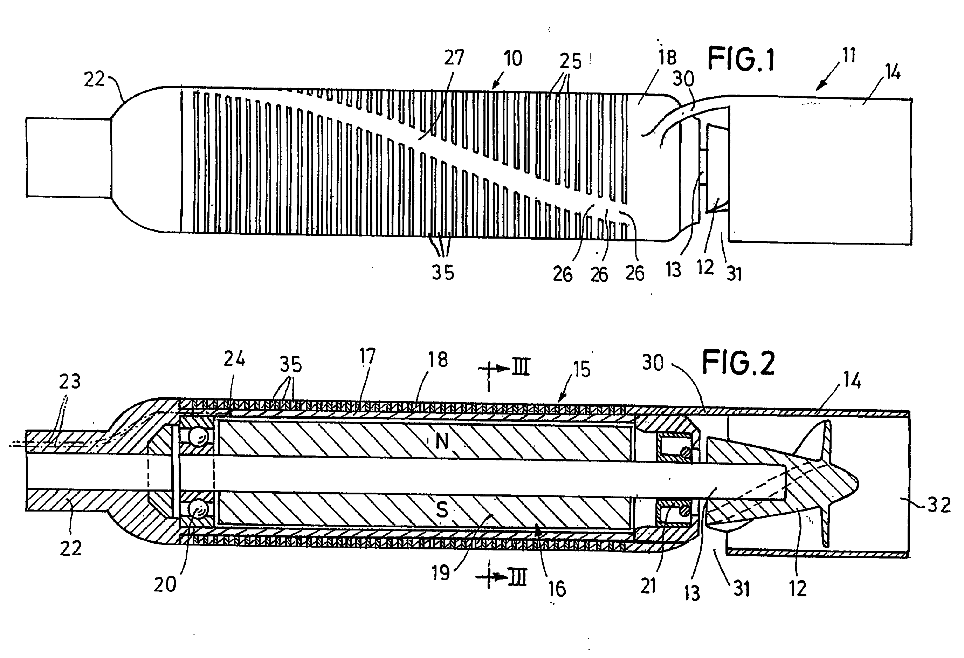

Der Rückschlussmantel 18 besteht aus einem einstückigen rohrförmigen Körper, in den umfangsmäßige Schlitze 25 eingeschnitten sind. Die Schlitze 25 begrenzen einzelne Ringe 35. Jeder Schlitz 25 erstreckt sich über weniger als 360°. Im vorliegenden Fall ist jeder Schlitz durch zwei einander diametral gegenüberliegende Brücken 26 unterbrochen. Die Brücken 26 benachbarter Schlitze bilden einen längslaufenden durchgehenden Steg 27. Dieser Steg verläuft hier schraubenförmig. Ein entsprechender Steg 27 befindet sich auf der in Fig. 1 nicht dargestellten gegenüberliegenden Seite.

Die Schlitze 25, die benachbarte Ringe 35 verbinden, sind durch Laserschneiden eines ferromagnetischen Rohres hergestellt, wobei die Schnittbreite weniger als ein zehntel Millimeter beträgt, insbesondere etwa 0,05 mm. Die Anzahl der Schlitze 25 sollte prinzipiell möglichst groß sein, so dass die Ringe so schmal wie möglich sind. Die Breite der Ringe beträgt vorzugsweise 0,2 - 0,3 mm.

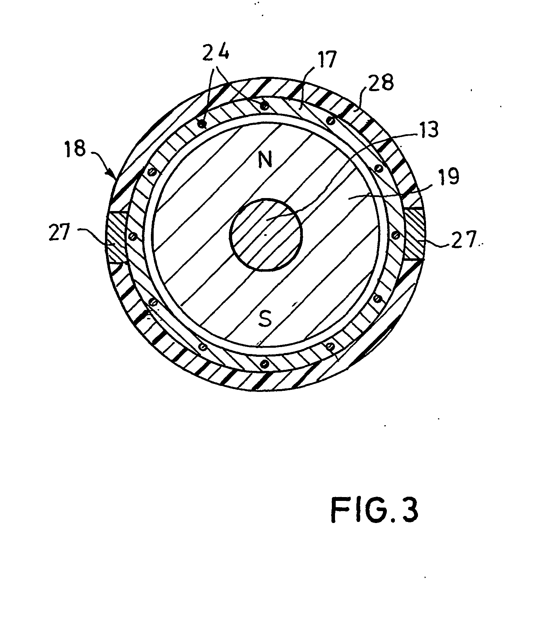

Die Schlitze 25 sind mit Kunststoff 28 ausgefüllt (Fig. 3), so dass sich eine geschlossene glatte Außenfläche des Rückschlussmantels 18 ergibt. Der Rückschlussmantel 18 bildet zugleich die Außenhaut des Mikromotors. Erforderlichenfalls kann er noch mit einer zusätzlichen Kunststoffschicht überzogen werden.

Wie Fig. 3 zeigt, erstrecken sich die mit dem Kunststoff 28 gefüllten Schlitze 25 durch die gesamte Stärke des Rückschlussmantels hindurch. Der Rückschlussmantel 18 umgibt eng die Hülse 17, welche die Erregerwicklung 24 enthält.

Die Wandstärke des Rückschlussmantels beträgt etwa 0,25 mm, der Außendurchmesser beträgt 4 mm und der Innendurchmesser 3,45. mm. Die Länge des Rückschlussmantels 18 beträgt 12 mm.

Der Rückschlussmantel 18 setzt sich durch Stege 30 fort, die vom vorderen Ende des Mikromotors nach vorne abstehen und einstückig in die Wand des Pumpengehäuses 14 übergehen. Das Pumpengehäuse 14 ist also einstückig mit dem Rückschlussmantel 18 hergestellt und besteht aus demselben Material. Dies bedeutet, dass das Pumpengehäuse 14 denselben Außendurchmesser und denselben Innendurchmesser hat wie der Rückschlussmantel 18. Die Stege 30 verlaufen bei dem vorliegenden Ausführungsbeispiel nicht parallel zur Achse des Mikromotors sondern schraubenförmig, entsprechend dem Verlauf der durch das schraubenförmige Flügelrad 12 erzeugten Strömung. Alternativ hierzu ist bei einem einstückig mit dem Rückschlussmantel hergestellten Pumpengehäuse die Wandstärke im Bereich des Pumpengehäuses geringer als im Bereich des Rückschlussmantels. So kann ein einheitliches Rohr vorgesehen sein, bei dem im Bereich des Pumpengehäuses der Innendurchmesser gegenüber dem Bereich des Rückschlussmantels durch Ausdrehen oder Bohren vergrößert ist, während der Außendurchmesser durchgehend gleich ist.

Die zwischen den Stegen 30 gebildeten Öffnungen 31 bilden die Auslassöffnungen der Pumpe und die stirnseitige Öffnung 32 des Pumpengehäuses 14 bildet die Einlassöffnung der Pumpe. Die Pumpe kann auch in Gegenrichtung angetrieben werden, so dass die Öffnungen 31 den Einlass und die Öffnung 32 den Auslass bilden.

## Patentansprüche

1. Mikromotor mit einem Stator (15), der eine Hülse (17) mit einer Erregerwicklung (24) und einen Rückschlussmantel (18) aus beabstandeten ferromagnetischen Ringen (35) aufweist,
wobei der Rückschlussmantel (18) aus einem einstückigen Körper besteht, bei dem benachbarte Ringe (35) durch mindestens eine Brücke (26) verbunden sind.

2. Mikromotor nach Anspruch 1, **dadurch gekennzeichnet, dass** die Brücken (26) einen längslaufenden durchgehenden Steg (27) bilden.

3. Mikromotor nach Anspruch 1 oder 2, **dadurch gekennzeichnet, dass** der Rückschlussmantel (18) einstückig in ein Pumpengehäuse (14) übergeht.

4. Mikromotor nach einem der Ansprüche 1 - 3, **dadurch gekennzeichnet, dass** die Lücken zwischen den Ringen mit einem Kunststoff (28) gefüllt sind, und dass der Rückschlussmantel (18) die Außenwand eines Motorgehäuses bildet.

5. Mikromotor nach einem der Ansprüche 1 - 4, **dadurch gekennzeichnet, dass** der Außendurchmesser des Rückschlussmantels (18) nicht mehr als 4 mm beträgt und der Innendurchmesser mindestens 3,3 mm beträgt.

6. Verfahren zum Herstellen eines Mikromotors, der eine Hülse (17) mit einer Erregerwicklung (24) und einen Rückschlussmantel (18) aus beabstandeten ferromagnetischen Ringen (35) aufweist, **dadurch gekennzeichnet, dass** der Rückschlussmantel (18) aus einem ferromagnetischen Rohr hergestellt wird, in welches durch Laserschneiden umlaufende, durch Brücken (26) unterbrochene Schlitze (25) eingeschnitten werden.

## Claims

1. A micromotor with a stator (15) comprising a sleeve (17) with an excitation winding (24) and an enveloping flux return structure (18) of spaced ferromagnetic rings (35),
wherein
the enveloping flux return structure (18) consists of an integral body wherein adjacent rings (35) are connected by at least one bridge (26).

2. The micromotor according to claim 1, **characterized in that** the bridges (26) form a longitudinal continuous web (27).

3. The micromotor according to claim 1 or 2, **characterized in that** the enveloping flux return structure (18) integrally passes into a pump housing (14).

4. The micromotor according to one of claims 1 to 3, **characterized in that** the gaps between the rings are filled with a plastic material (28), and that the enveloping flux return structure (18) forms the outer wall of a motor housing.

5. The micromotor according to one of claims 1 to 4, **characterized in that** the outer diameter of the enveloping flux return structure (18) does not amount to more than 4 mm and that the inner diameter amounts to at least 3.3 mm.

6. A method for manufacturing a micromotor comprising a sleeve (17) with an excitation winding (24) and an enveloping flux return structure (18) of spaced ferromagnetic rings (35), **characterized in that** the enveloping flux return structure (18) is made of a ferromagnetic tube in which peripheral slots (25) interrupted by bridges (26) are cut by laser cutting.

## Revendications

1. Micromoteur avec un stator (15), comportant une douille (17) avec un enroulement inducteur (24) et une culasse de reflux (18) en anneaux ferromagnétiques (35) écartés,
la culasse de reflux (18) étant en un corps monobloc sur lequel des anneaux voisins (35) sont reliés par au moins un pont (26).

2. Micromoteur selon la revendication 1, **caractérisé en ce que** les ponts (26) forment une entretoise (27) continue s'étendant en longueur.

3. Micromoteur selon la revendication 1 ou 2, **caractérisé en ce que** la culasse de reflux (18) passe en monobloc dans un carter de pompe (14).

4. Micromoteur selon l'une des revendications 1 à 3, **caractérisé en ce que** les interstices entre les anneaux sont remplis d'une matière plastique (28) et **en ce que** la culasse de reflux (18) forme la paroi extérieure d'un carter de moteur.

5. Micromoteur selon l'une des revendications 1 à 4, **caractérisé en ce que** le diamètre extérieur de la culasse de reflux (18) n'est pas supérieur à 4 mm et **en ce que** le diamètre intérieur est d'au moins 3,3 mm.

6. Procédé de fabrication d'un micromoteur, comportant une douille (17) avec un enroulement inducteur (24) et une culasse de reflux (18) en anneaux ferromagnétiques écartés (35), **caractérisé en ce qu'**on fabrique la culasse de reflux (18) en un tube ferromagnétique, dans lequel par découpe au laser, on incise des entailles périphériques (25), interrompues par des ponts (26).
